# EUROPEAN PATENT APPLICATION

(11) **EP 2 676 655 A1**
(43) Date of publication of application: **25.12.2013**
(21) Application number: 13172472.6
(22) Date of filing: 18.06.2013
(51) Int. Cl.: A61J 19/00, B65F 1/00, A61F 5/44, B65D 33/16

(54) **A bag**

(30) Priority: 18.06.2012 TR 201207070
(71) Applicant: EGE Universitesi, 35100 Izmir (TR)
(72) Inventor: Bozdag, Ali Dogan, 35280 Izmir (TR)
(74) Representative: Dericioglu Kurt, Ekin

(57) **Abstract**

The present invention relates to a vomiting bag (1) which can be easily opened and closed by a single hand at the time of vomiting. The inventive bag (1) comprises at least one half ring (3) which is in the shape of a circular angle of 180° relative to a linear plane and which includes at least one protrusion on the outer surface thereof; at least one preferably transparent chamber (8) which is located below the half rings (3), and into which a vomiting person discharges her/his vomit; at least one circular mouth (2) which is formed by connection of two half rings (3) and which can be opened and closed; at least one rod (4) which connects the half rings (3) to each other and which passes from the center of the mouth (2).

## Description

### Field of the Invention

The present invention relates to a vomiting bag which can be easily opened and closed at the time of vomiting.

### Background of the Invention

There are vomiting bags in the state of the art for getting vomiting under control. These bags are generally disposable and are discarded after use. Inner part of the bags is comprised of a sealing material. A material, which absorbs the liquid and forms a thick viscosity and thus prevents spillage, is also added in some bags.

Outside of the bag may be coated with a thicker material similar to cardboard. By means of this harder material, the bag is straighter and it is easier to open and close the mouth of the bag. When needed, the bag is taken from its place, its mouth is opened, the need is satisfied, then its mouth is closed and it is thrown away.

There are various kinds of the current vomiting bags and they have different properties relative to the area of use. They are practical and suitable for use in passenger vehicles. Special containers and vomiting bags are used in hospitals. The bags used in hospitals are generally made of a transparent material. The type of bags that can be hung around the neck may be more suitable for use by the patients.

In case of need, the patient takes the bag which is nearby, opens it and after using, closes and throws it away. In case of the models that are hung around the neck, the patient or her/his attendant opens the bag which is hung around the patient's neck, closes it after use, and removes it from the neck and throws it away. Regardless of the type or model of the bag, it requires use of both hands, i.e. while one hand is holding the bag, the other hand opens the bag, moves it towards the mouth and the need is satisfied. The current bags are closed and thrown away after use by using two hands.

If there is sufficient time for opening the bag and closing it after use when necessary, it is not a problem; but in case of emergencies there might not be enough time even to grab and open the bag. The type of bags that are hung around the neck may be more useful, but nevertheless it may not always be easy to open the bag, move it towards the mouth and vomit into the bag without polluting the environment in spite of use of two hands.

In cases where the patient cannot use one of her/his hands, it becomes fairly difficult for the bag to fulfill its function. As a matter of fact, even the patient attendant attending the patient might not be able to reach at the time of vomiting of the patient. Because she/he may be late to open the bag and hold it towards the patient's mouth. The same is applicable for mothers with babies. The currently used bags are generally insufficient when babies vomit. A mother feeding her baby on a high chair should keep a container or a bag available by estimating in advance the time that her baby would vomit, otherwise she might be too late.

Chinese utility model document no. CN2257808 (Y), known in the state of the art, discloses a multifunctional cleaning bag composed of a bag, frames, and handles. There is an opening on one end of the bag. There are two semi-circular frames on two ends of this opening. One of these frames is concave while the other is convex. These two semicircles are mutually engaged. Both sides of each semicircle are connected to each other through a movable shaft. The frames, which are connected to each other, can be opened and closed.

Chinese utility model document no. CN201329043 (Y), known in the state of the art, discloses a bag which has a circumferential sealing elastic tape and which is used during vomiting of patients. The bag is used during vomiting by attaching the ferrules of the bag to the ears of the patient.

Chinese utility model document no. CN201329044 (Y), known in the state of the art, discloses a bag which is mostly used for bedridden patients. Bag noses are arranged on the opening of the bag and a loop band passes through the bag noses.

### Summary of the Invention

The objective of the present invention is to provide a bag which can be opened and closed by a single hand after being tied around the neck.

Another objective of the present invention is to provide a bag which has a spring mechanism that is opened when the mouth part is held and pulled, and is closed when the mouth part is released.

A further objective of the present invention is to provide a bag which can be closed by itself by means of the spring.

Another objective of the present invention is to provide a bag which will provide maximum facility for the user to use it by herself/himself.

### Detailed Description of the Invention

A bag developed to fulfill the objective of the present invention is illustrated in the accompanying figures wherein,

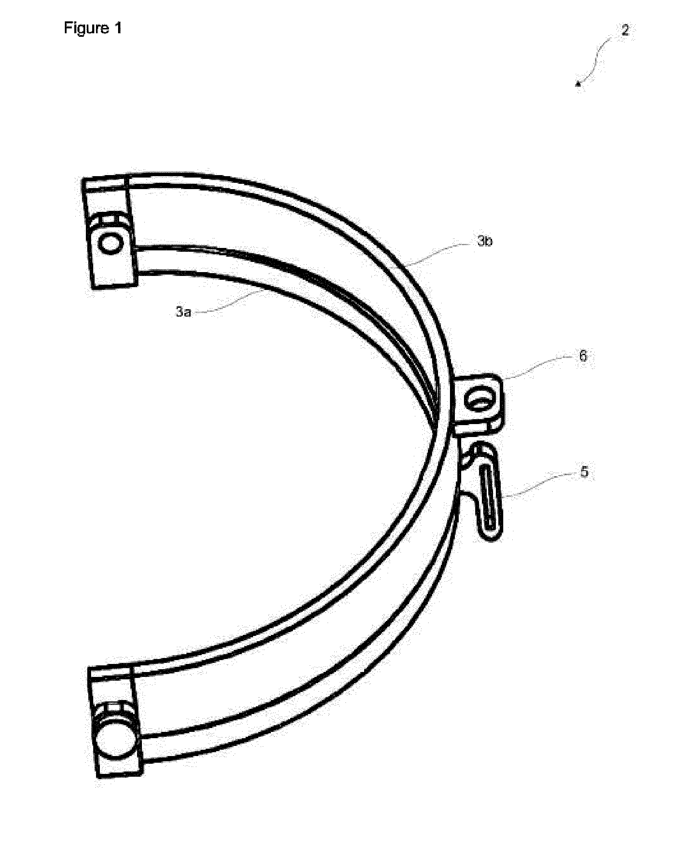

Figure 1 is the perspective view of the mouth in closed position.

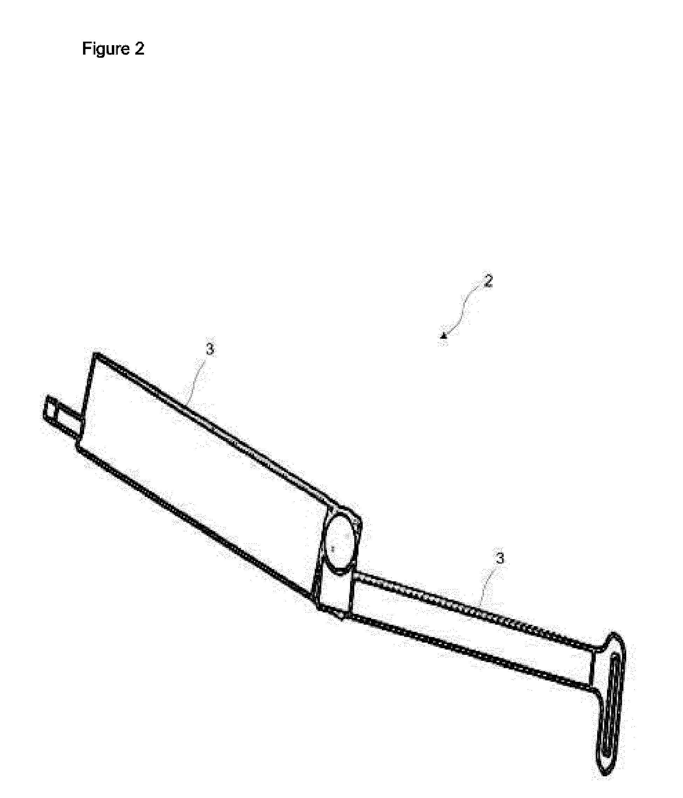

Figure 2 is the side view of the mouth in open position.

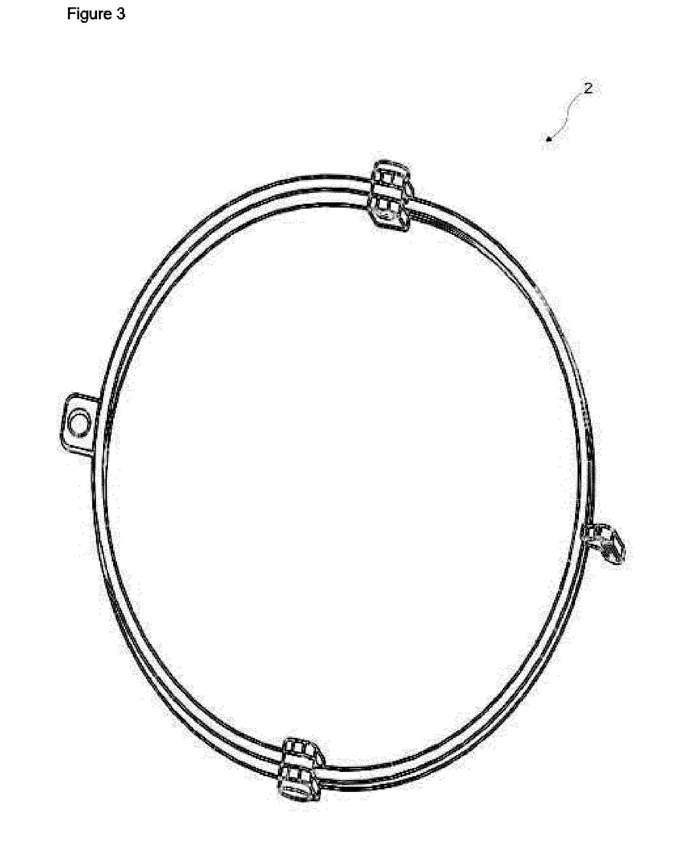

Figure 3 is the top view of the mouth in open position.

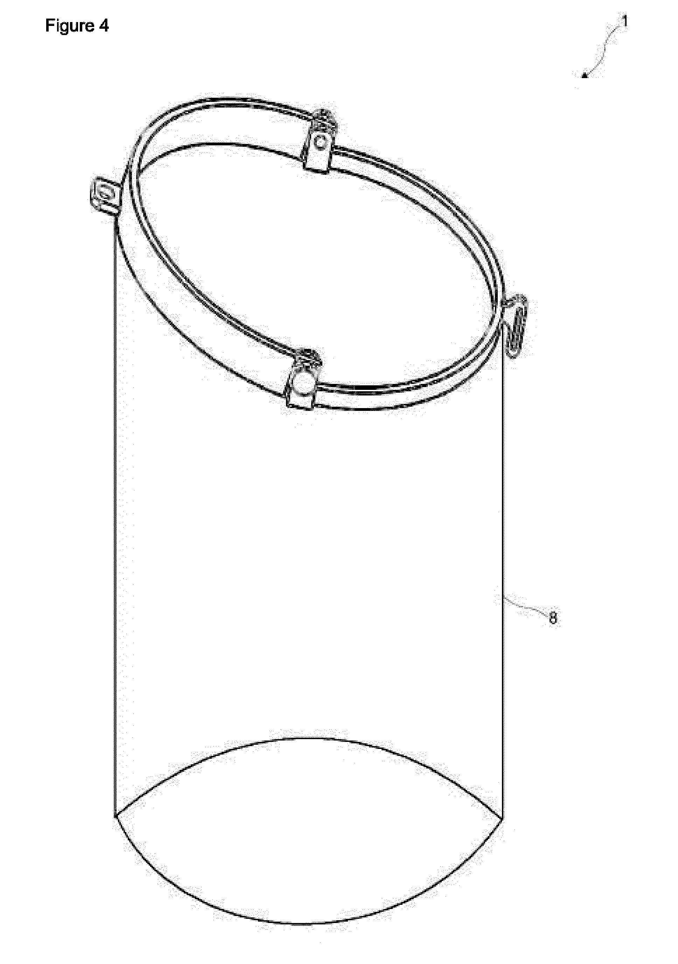

Figure 4 is the perspective view of the bag.

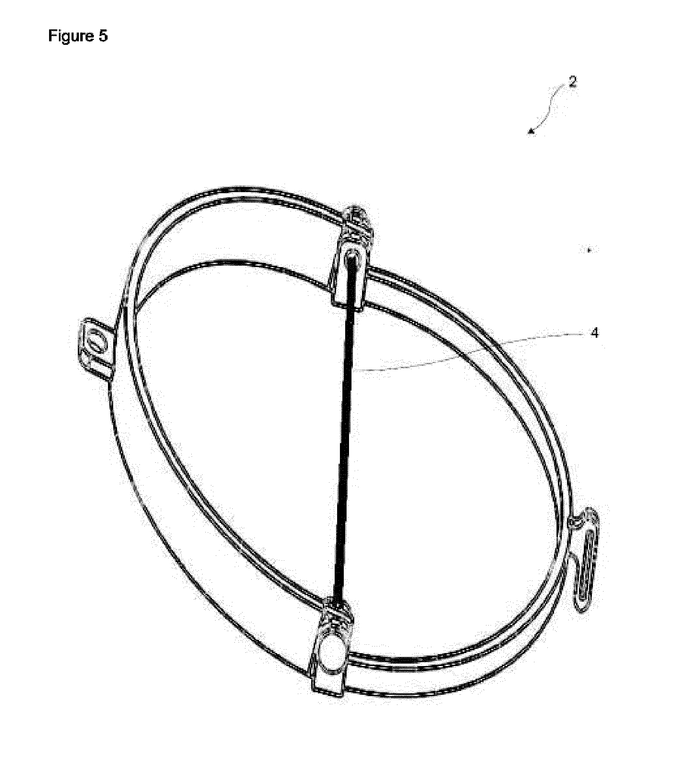

Figure 5 is the perspective view of the mouth in open position where the rod is visible.

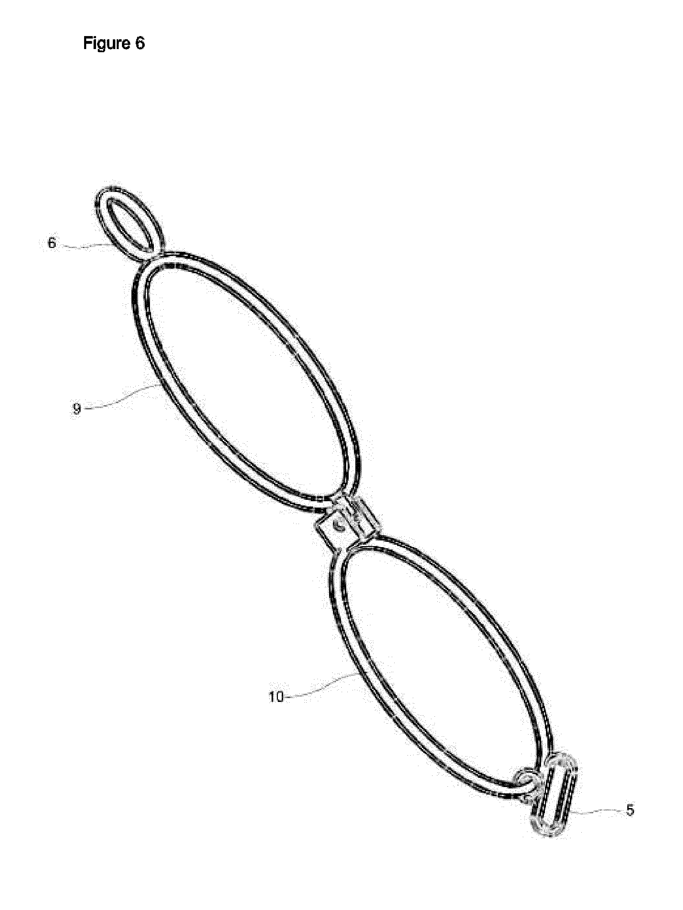

Figure 6 is the open perspective view of the full rings in the preferred embodiment of the invention.

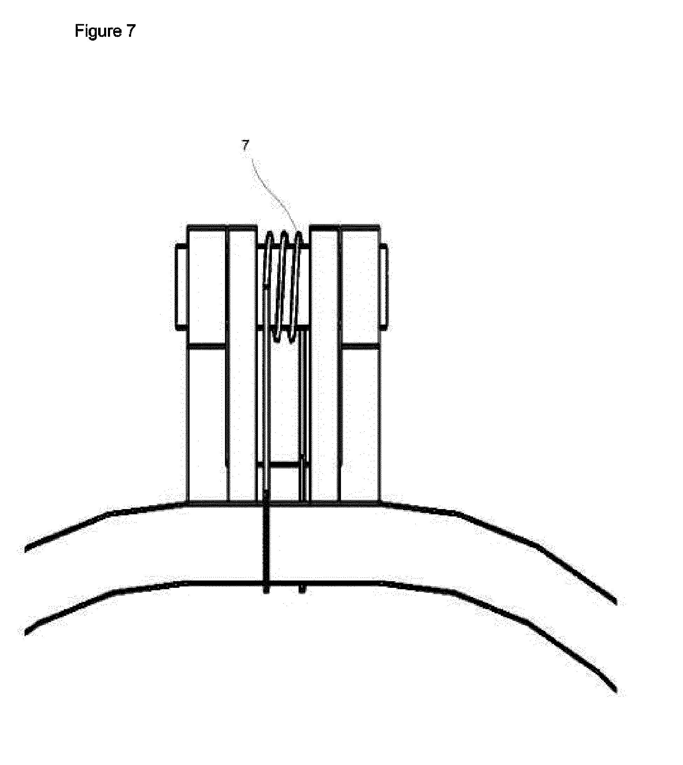

Figure 7 is the front view of the spring.

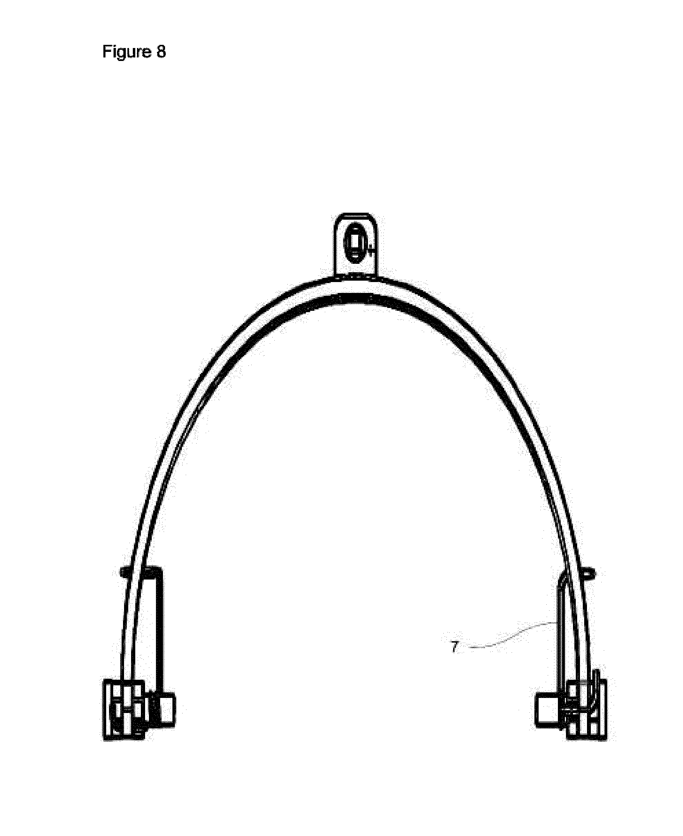

Figure 8 is the top view of the mouth in closed position wherein the spring is visible.

The components shown in the figures are each given reference numerals as follows:
1. Bag
2. Mouth
3. Half ring
   3.a. Primary half ring
   3.b. Secondary half ring
4. Rod
5. Hollow protrusion
6. Handle
7. Spring
8. Chamber
9. Primary full ring
10. Secondary full ring

A bag (1), which can be easily opened and closed by using a single hand at the time of vomiting, basically comprises
- at least one half ring (3) which is in the shape of a circular angle of 180° relative to a linear plane and which includes at least one protrusion on the outer surface thereof,
- at least one preferably transparent chamber (8) with horizontal scaling lines thereon which is located below the half rings (3), and into which a vomiting person discharges her/his vomit,
- at least one circular mouth (2) which is formed by connection of two half rings (3) and which can be opened and closed,
- at least one rod (4) which connects the half rings (3) to each other and which passes from the center of the mouth (2),
- at least one primary half ring (3a) which is connected to the rod (4),
- at least one secondary half ring (3b) which is connected to the rod (4),
- at least one hollow protrusion (5) which is positioned on the primary half ring (3a) and which includes a circular hollow,
- at least one elliptical handle (6) which is positioned on the secondary half ring (3b) and which includes a hollow,
- at least one spring (7) which enables the half rings to be positioned tight on top of each other and which enables them to be opened and closed.

Vomiting is ejecting the contents of the stomach through the mouth. Generally it occurs fast and if it is not under control; clothes, surrounding and the objects around the person get dirty. This situation is very disturbing both visually and because of the bad odor and it also embarrasses the person.

A feeling of nausea may occur before vomiting and in this case, a container or a bag is immediately sought or one tries to reach a lavatory urgently. Although there is this chance at home, it diminishes when one is outside.

On the upper part of the inventive bag (1), there is a circular mouth (2) which is formed by connection of two half rings (3) that can be folded on each other. There is a rod (4) that passes from the center of this mouth (2). The circular mouth (2) is folded into two over the rod (4) and turns into two nested half rings (3). There is provided a spring (7) at each end where the rod (4) is connected with the half rings (3).

When the mouth (2) of the bag (1) is opened by pulling, the springs (7) are biased, and when the mouth is released, the springs (7) return to their initial position and in the meantime the mouth (2) of the bag (1) is closed. When the half rings (3), which open the mouth (2) of the bag (1) by forming a circle when pulled by the springs (7), are released, they fold one on top of another and close the mouth (2) of the bag (1). There are protrusions on the edges of the half rings (3).

A band is passed though the hollow protrusion (5) provided on the outer side of the primary half ring (3) and the bag is tied with the band around the neck. The other protrusion is a handle (6) and it is used for closing the mouth (2) of the bag (1) upon being pulled. When the handle (6) is released while the mouth (2) of the bag (1) is open, the biased springs (7) return to their original position and the half rings (3) fold one on top of another and close the mouth (2) of the bag (1).

A chamber (8) is connected to both half rings (3) and when they form a circle, the mouth (2) of the chamber (8) becomes open. When the circle turns into two nested half rings (3), the mouth (2) of the bag (1) is closed. The chamber (8) is opened by holding and pulling upwards the handle (6) of the bag (1) that is hung around the neck, and it comes to level with the lips. Thus, the bag (1) comes to level with the lips by using a single hand and with a single movement.

The mouth (2) of the bag (1), which is open as long as its handle (6) is kept pulled, closes when its handle (6) is released, and thus use of a second hand or any other efforts are not required. As the mouth (2) of the bag (1) is normally closed, it prevents the odor from getting out, therefore it does not have to be thrown away after a single use, it allows to be used several times. The chamber (8) is preferably transparent and includes scales thereon, and thus also provides information regarding the content, color, viscosity and amount of the vomit.

In the preferred embodiment of the invention, the mouth (2) is comprised of two full rings instead of half rings (3). The primary full ring (9) and the secondary full ring (10) are connected to each other. The secondary full ring (10) is located below the primary full ring (9) and the chamber (8) is connected to the secondary full ring (10). The primary full ring (9) can be opened and closed. Thus, when the primary full ring (9) is held and opened by being pulled upwards, the secondary full ring (10) and the chamber (8) come to level with the lips and enable the vomit to be ejected into the chamber (8) and then upon being closed prevents odor of the vomit from getting out to the outer environment.

In another preferred embodiment of the invention, the mouth (2) is comprised of a rubber that can stretch, instead of half rings (3). There is provided a pulling ring on the rubber and thus the mouth (2) can be easily opened. Additionally the chamber (8) can be made of a soundproof material and thus propagation of a part of the sound waves that will occur during vomiting can be prevented. Additionally, by means of a lock mechanism, which can be opened and closed and is positioned to a point near the mouth (2) of the chamber (8), the odor of the vomit is prevented from spreading to the outer environment.

Within the framework of these basic concepts, it is possible to develop a wide variety of embodiments of the inventive bag (1). The invention can not be limited to the examples described herein and it is essentially as defined in the claims.

## Claims

1. A bag (1), which can be easily opened and closed by using a single hand at the time of vomiting, basically **comprising**
- at least one preferably transparent chamber (8) with horizontal scaling lines thereon which is located below the half rings (3), and into which a vomiting person discharges her/his vomit,
- at least one primary half ring (3a) which is connected to the rod (4),
- at least one secondary half ring (3b) which is connected to the rod (4),
- at least one spring (7) which enables the half rings to be positioned tight on top of each other and which enables them to be opened and closed,
**and characterized by**
- at least one circular mouth (2) which is formed by connection of two half rings (3) and which can be opened and closed,
- at least one rod (4) which connects the half rings (3) to each other and which passes from the center of the mouth (2),
- at least one elliptical handle (6) which is positioned on the secondary half ring (3b) and which includes a hollow,
- at least one hollow protrusion (5) which is positioned on the primary half ring (3a) and which includes a circular hollow,
- at least one half ring (3) which is in the shape of a circular angle of 180° relative to a linear plane and which includes at least one protrusion on the outer surface thereof.

2. A bag (1) according to Claim 1, **characterized by** at least one circular mouth (2) which is formed by connection of two half rings (3) that can be folded on each other.

3. A bag (1) according to Claim 1 and 2, **characterized by** the circular mouth (2) which turns into two nested half rings (3) by being folded into two over the rod (4).

4. A bag (1) according to any one of the preceding claims, **characterized by** the spring (7) which is provided at each end where the rod (4) is connected with the half rings (3).

5. A bag (1) according to any one of the preceding claims, **characterized by** at least one spring (7) which is biased when the mouth (2) is opened by being pulled.

6. A bag (1) according to any one of the preceding claims, **characterized by** at least one primary half ring (3a) including a hollow protrusion (5) through which a band is passed to tie the bag around the neck.

7. A bag (1) according to any one of the preceding claims, **characterized by** the half ring (3) which closes the mouth (2) of the bag (1) by being folded one on top of another.

8. A bag (1) according to any one of the preceding claims, **characterized by** at least one transparent chamber (8) which is connected to both of the half rings (3).

9. A bag (1) according to any one of the preceding claims, **characterized by** at least one mouth (2) which, when it is closed, prevents the odor from getting out to the outer environment.

10. A bag (1) according to any one of the preceding claims, **characterized by** at least one handle (6) which opens the mouth (2) of the chamber as long as it is kept pulled.

11. A bag (1) according to any one of the preceding claims, **characterized by** at least one chamber (8) which is preferably transparent and includes scales thereon, and which also provides information regarding the content, color, viscosity and amount of the vomit.
